# EUROPÄISCHE PATENTSCHRIFT

(11) **EP 1 788 960 B1**
(45) Veröffentlichungstag und Bekanntmachung des Hinweises auf die Patenterteilung: **17.12.2008**
(21) Anmeldenummer: 05777138.8
(22) Anmeldetag: 24.08.2005
(51) Int. Cl.: A61B 17/29

(54) **MEDIZINISCHES SCHNEID-UND/ODER HALTEINSTRUMENT**
MEDICAL CUTTING AND/OR HOLDING INSTRUMENT
INSTRUMENT MEDICAL DE COUPE ET/OU DE MAINTIEN

(30) Priorität: 25.08.2004 DE 102004041080
(43) Veröffentlichungstag der Anmeldung: 30.05.2007
(73) Patentinhaber: Karl Storz GmbH & Co. KG, 78532 Tuttlingen (DE)
(72) Erfinder: STORZ, Martin, 78532 Tuttlingen (DE)
(74) Vertreter: Hofmeister, Frank
(86) Internationale Anmeldenummer: PCT/EP2005/009130
(87) Internationale Veröffentlichungsnummer: WO 2006/021432

(56) Entgegenhaltungen:
- WO-A-20/05079680
- DE-U1-6202004 000 27

## Beschreibung

Die Erfindung betrifft ein medizinisches Schneid- und/oder Halteinstrument mit einem Schaft, einem am distalen Ende des Schaftes angeordneten, aus zwei Maulteilen bestehenden Werkzeug sowie mit einer am proximalen Ende des Schaftes angeordneten, aus zwei Griffteilen bestehenden Handhabe zum Öffnen und Schließen der Maulteile des Werkzeugs.

Als Schaftinstrumente ausgebildete medizinisches Schneid- und/oder Halteinstrumente sind aus der Praxis in den verschiedensten Ausführungsformen bekannt. Die aus der Praxis bekannten Schaftinstrumente weisen sowohl maulseitig, also distal als auch handhabenseitig, also proximal Gelenkkonstruktionen auf, welche es erlauben, über die Betätigung eines verschwenkbaren Griffteils der Handhabe die Maulteile des Werkzeugs zum Öffnen und Schließen gegeneinander zu verschwenken. Das Betätigen der Maulteile über den verschwenkbaren Griffteil erfolgt dabei in der Regel über eine in dem hohlen Instrumentenschaft verschiebbar gelagerte Schub-/Zugstange.

Nachteilig bei diesen bekannten als Schaftinstrumente ausgebildeten medizinischen Schneid- und/oder Halteinstrumenten ist, dass sowohl ihre Maulmechanik als auch die Mechanik der gegeneinander verschwenkbaren Griffteile der Handhabe herstellungstechnisch aufwendige Gelenkkonstruktionen erfordern. Neben diesem hohen Bauteil- und Montageaufwand ist es bei den bekannten Hohlschaftinstrumenten zwingend erforderlich, dass diese zerlegbar ausgebildet sind, um eine einwandfreie Reinigung des Instruments zu gewährleisten.

Daneben ist aus der Gebrauchsmusterschrift DE 20 2004 000 276 U1 ein chirurgisches Mikroinstrument bekannt, das die Merkmale des Oberbegriffs des Anspruchs 1 aufweist.

Die nachveröffentlichte Patentanmeldung WO 2005/079680 A2 schließlich beschreibt ein medizinisches Schneid- und/oder Halteinstrument, das sich vom Gegenstand des Anspruchs 1 dadurch unterscheidet, dass die Schaftstangen nicht einstückig mit je einem der Griffteile ausgebildet sind.

Davon ausgehend liegt der Erfindung die **Aufgabe** zugrunde, ein medizinisches Schneid- und/oder Halteinstrument der eingangs genannten Art so auszugestalten, dass es einfach aufgebaut und gut zu reinigen ist.

Die **Lösung** dieser Aufgabenstellung ist erfindungsgemäß in Anspruch 1 definiert.

Durch die gelenklose einstückige Ausbildung von Schaftstange und Griffteil der Handhabe kann im Bereich der Handhabe ganz auf eine Gelenkmechanik verzichtet werden. Darüber hinaus wird die Zahl der einzelnen Bauteile gegenüber den bekannten Hohlschaftinstrumenten deutlich reduziert, wodurch eine einfachere und kostengünstigere Montage ermöglicht wird.

Gemäß einer ersten praktischen Ausführungsform der Erfindung wird vorgeschlagen, dass die Griffteile der Handhabe über ein Kopplungselement miteinander verbunden sind. Über dieses Kopplungselement werden die Griffteile der Handhabe zu einer Einheit zusammengefasst, wodurch die Handhabung des Instruments vereinfacht werden kann.

Um die Anzahl der einzelnen Bauteile noch weiter zu reduzieren, wird gemäß einer bevorzugten Ausführungsform der Erfindung vorgeschlagen, dass die die beiden Griffteile aufweisende Handhabe als einstückiges Bauteil ausgebildet ist. Bei dieser Konstruktion bilden zumindest die beiden Schaftstangen und die beiden Griffteile der Handhabe nur ein einziges Bauteil. Ein solche vorzugsweise aus vorgefertigtem Stangenmaterial hergestellte umlaufende Rahmenkonstruktion ist einfach und kostengünstig zu fertigen und darüber hinaus auch ohne Demontage zuverlässig zu reinigen.

Das Betätigen der Maulteile des Werkzeugs über die Griffteile der Handhabe erfolgt erfindungsgemäß dadurch, dass die Griffteile der Handhabe federelastisch relativ zueinander verlagerbar sind. Die Federelastizität der vorzugsweise aus Edelstahl gefertigten Griffteile ersetzt bei der erfindungsgemäßen Konstruktion die aufwendige Gelenkmechanik der bekannten Griffkonstruktionen.

Um dem Operateur das Ergreifen der Handhabe zu erleichtern und eine sichere und präzise Bedienung eines erfindungsgemäßen medizinischen Schneid- und/oder Halteinstruments zu gewährleisten, ist gemäß einer ersten Ausführungsform der Erfindung die Handhabe zumindest im Bereich eines Griffteils abgeflacht ausgebildet.

Alternativ wird erfindungsgemäß vorgeschlagen, dass an mindestens einem Griffteil der Handhabe mindestens eine Fingermulde ausgebildet ist oder an mindestens einem Griffteil der Handhabe eine Griffplatte festlegbar ist, um ein lagesicheres Ergreifen der Handhabe zu ermöglichen.

Weiterhin wird mit der Erfindung vorgeschlagen, dass ein Maulteil des Werkzeugs einstückig starr mit einer Schaftstange ausgebildet ist und das andere Maulteil um Anlenkpunkte verschwenkbar an der anderen Schaftstange sowie am starren Maulteil gelagert ist. Durch die einstückig starre Ausbildung des einen Maulteils wird die Anzahl der Einzelteile weiter reduziert. Vorzugsweise ist die erfindungsgemäße Maulkonstruktion so ausgebildet, dass unterschiedliche Übersetzungsverhältnisse erzielbar sind. Hierzu wird vorgeschlagen, dass der Abstand der Anlenkpunkte des verschwenkbaren Maulteils veränderbar ist.

Insbesondere für die Ausgestaltung des erfindungsgemäßen medizinischen Instruments als Halteinstrument sind die Griffteile der Handhabe und/oder die Schaftstangen zumindest in einer Schließstellung des Werkzeugs über einen Arretiermechanismus in ihrer jeweiligen Lage zueinander fixierbar.

Neben der Verwendung der erfindungsgemäßen medizinischen Instrumente als bloße Schneid- und/oder Halteinstrumente sind diese auch so ausgestaltbar, dass diese als monopolares oder bipolares Instrument für die Elektrochirurgie verwendbar sind. Bei der sogenannten Elektrochirurgie wird dem Operationsgebiet über das medizinische Instrument Hochfrequenzenergie zugeführt, die im Gewebe in Wärmeenergie umgewandelt wird, wobei am Umwandlungsort Temperaturen von etwa 60°C bis 100°C entstehen. Diese Elektrochirurgie wird zum Koagulieren von Gewebe und kleinen Gefäßen sowie zum Schneiden oder Trennen von Gewebe eingesetzt.

Zur Verwendung eines erfindungsgemäßen medizinischen Instruments in der Elektrochirurgie wird vorgeschlagen, dass die Griffteile der Handhabe mit einem Verbindungsstecker verbunden sind, über den der Hochfrequenzstrom in das Instrument eingeleitet und bei der bipolaren Anwendung auch wieder abgeleitet wird.

Bei der Ausbildung der Handhabe mit zwei separaten Griffteilen wird erfindungsgemäß vorgeschlagen, dass der Verbindungsstecker als die beiden Griffteile der Handhabe miteinander verbindendes Kopplungselement ausgebildet ist.

Die getrennt voneinander verlaufenden Schaftstangen mit den einstückig gelenklos daran angeformten Griffteilen der Handhabe bieten ausgesprochen günstige Isolationsbedingungen bei der Nutzung als Zu- und Ableitung des elektrischen Hochfrequenzstroms. Hierzu wird gemäß einer praktischen Ausführungsform der Erfindung vorgeschlagen, dass bis auf die beiden Maulteile des Werkzeugs alle stromführenden Teile; insbesondere mittels eines Schrumpfschlauches, elektrisch isoliert ausgebildet sind.

Um eine vorzeitige Überleitung des Stroms in die stromableitende Schaftstange zu verhindern, wird mit der Erfindung schließlich vorgeschlagen, dass das verschwenkbare Maulteil über eine elektrisch isolierte Gelenklasche am starren Maulteil und an der Schaftstange gelagert ist.

Weitere Merkmale und Vorteile der Erfindung ergeben sich anhand der zugehörigen Zeichnung, in der sechs Ausführungsbeispiele eines erfindungsgemäßen medizinischen Schneid- und/oder Halteinstruments nur beispielhaft dargestellt sind. In der Zeichnung zeigt:
- Fig. 1: eine Seitenansicht einer ersten Ausführungsform eines erfindungsge- mäßen medizinischen Schneid- und/oder Halteinstruments;
- Fig. 2: eine Seitenansicht einer zweiten Ausführungsform eines erfindungsge- mäßen medizinischen Schneid- und/oder Halteinstruments;
- Fig. 3: eine Seitenansicht einer dritten Ausführungsform eines erfindungsge- mäßen medizinischen Schneid- und/oder Halteinstruments;
- Fig. 4a: eine Seitenansicht einer vierten Ausführungsform eines erfindungsge- mäßen medizinischen Schneid- und/oder Halteinstruments;
- Fig. 4b: eine Rückansicht des Instruments gemäß Fig. 4a;
- Fig. 4c: eine vergrößerte Detailansicht des Details IVc gemäß Fig. 4a;
- Fig. 4d: eine Draufsicht auf die Darstellung gemäß Fig. 4c,
- Fig. 5: eine Seitenansicht einer fünften Ausführungsform eines erfindungsge- mäßen medizinischen Schneid- und/oder Halteinstruments;
- Fig. 6a: eine Seitenansicht einer sechsten Ausführungsform eines erfindungs- gemäßen medizinischen Schneid- und/oder Halteinstruments;
- Fig. 6b: eine vergrößerte und teilweise geschnittene ausschnittweise Seitenan- sicht des Instruments gemäß Fig. 6a und
- Fig. 6c: einen Schnitt entlang der Linie Vlc-Vlc gemäß Fig. 6b.

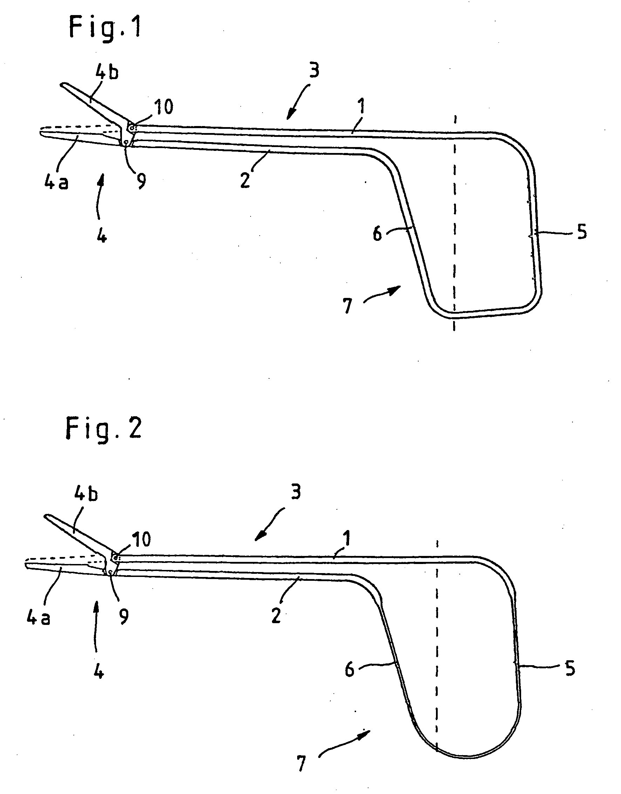

Die in den Abbildungen Fig. 1, 2, 3, 4a, 5 und 6a in der Seitenansicht dargestellten medizinischen Schneid- und/oder Halteinstrumente bestehen im Wesentlichen aus einem aus zwei separaten Schaftstangen 1 und 2 gebildeten Schaft 3, einem am distalen Ende des Schaftes 3 angeordneten, aus zwei Maulteilen 4a und 4b bestehenden Werkzeug 4 sowie einer am proximalen Ende des Schaftes 3 angeordneten, aus zwei Griffteilen 5 und 6 bestehenden Handhabe 7.

Die in den Abbildungen dargestellten medizinischen Schneid- und/oder Halteinstrumente weisen die Besonderheit auf, dass jedes Griffteil 5 und 6 der Handhabe 7 und die jeweils zugehörige Schaftstange 1 und 2 des Schaftes 3 einstückig gelenklos ausgebildet sind.

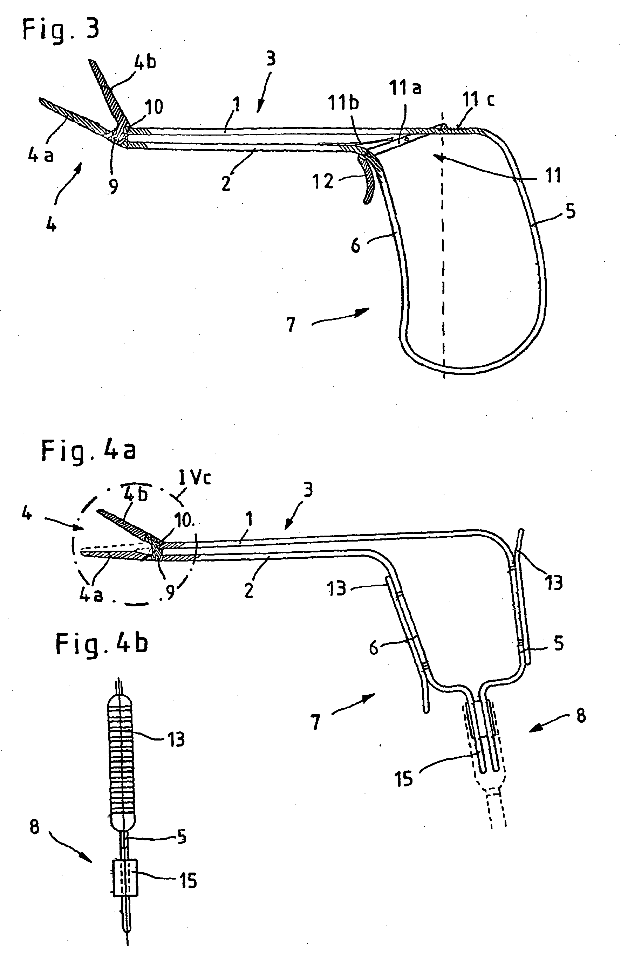

Weiterhin lassen sich die dargestellten Instrumente in zwei Gruppen mit unter schiedlich ausgebildeten Handhaben 7 unterteilen, nämlich einerseits die in den Abbildungen Fig. 1, 2 und 3 dargestellten Instrumente, bei denen auch die Griffteile 5 und 6 der Handhabe 7 gelenklos miteinander verbunden ein einstückiges Bauteil bilden und andererseits die in den Abbildungen Fig. 4a, 5 und 6a dargestellten Instrumente, bei denen die Griffteile 5 und 6 der Handhabe 7 über ein Kopplungselement 8 gelenklos miteinander verbunden sind.

In allen Fällen zeichnen sich die dargestellten medizinischen Schneid- und/oder Halteinstrumente dadurch aus, dass sie aus nur wenigen Bauteilen bestehen und im Bereich der Handhabe 7 vollständig auf eine aufwendige Gelenkmechanik verzichtet wurde.

Das Öffnen und Schließen der Maulteile 4a und 4b des Werkzeugs 4 über die Handhabe 7 erfolgt bei den dargestellten medizinischen Instrumenten dadurch, dass die Griffteile 5 und 6 der entweder einstückig hergestellten Handhabe 7 gemäß Fig. 1 bis 3 oder aber die über das Kopplungselement 8 miteinander verbundenen Griffteile 5 und 6 der Handhabe 7 gemäß Fig. 4a bis 6a federelastisch relativ zueinander verlagerbar ausgebildet sind. Die vorzugsweise aus rundem oder flachem Edelstahl gefertigten und einstückig mit den jeweiligen Schaftstangen 1 und 2 des Schaftes 3 verbundenen Griffteile 5 und 6 sind dabei so ausgebildet, dass beim Umgreifen der Handhabe 7 der Griffteil 5 im Wesentlichen starr bleibt und der Griffteil 6 durch das Anziehen der Finger an das starre Griffteil 6 heranziehbar ist. Aufgrund der Federelastizität des Materials der miteinander verbundenen Griffteile 5 und 6 federt der Griffteil 6 sofort wieder in die dargestellte Ausgangsstellung zurück.

Aufgrund der einstückigen Ausbildung der Griffteile 5 und 6 der Handhabe 7 mit jeweils einer Schaftstange 1 und 2 des Schaftes 3 ergeben sich somit eine im Wesentlichen starre Schaftstange 1, die einstückig mit dem starren Griffteil 5 ausgebildet ist, sowie eine im Wesentlichen axial verlagerbaren Schaftstange 2, die einstückig mit dem flexibel verlagerbaren Griffteil 6 ausgebildet ist.

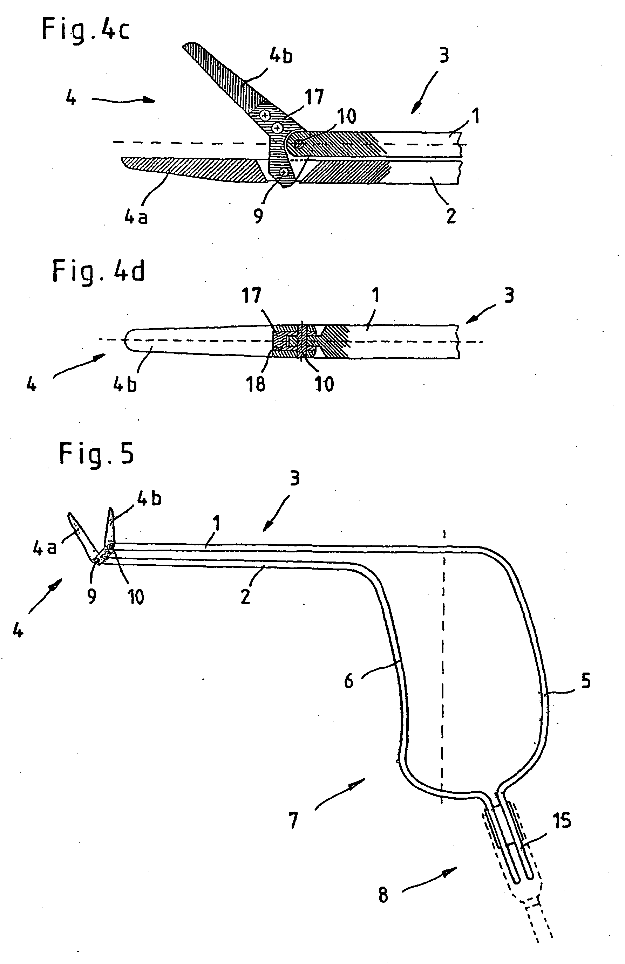

Wie weiterhin aus den Seitenansichten der verschiedenen Instrumente ersichtlich, ist das Maulteil 4a des Werkzeugs 4 einstückig starr mit Schaftstange 2 ausgebildet, die ihrerseits einstückig mit dem flexibel verlagerbaren Griffteil 6 ausgebildet ist. Der Aufbau des Werkzeugs 4 mit einem starren Maulteil 4a und einem verschwenkbaren Maulteil 4b ist insbesondere der Abbildung Fig. 4c zu entnehmen.

Das als Kipphebel ausgebildete verschwenkbare Maulteil 4b ist um Anlenkpunkte 9 und 10 verschwenkbar an der starren Schaftstange 1 und dem starren Maulteil 4a gelagert, wobei die Anlenkpunkte 9 und 10 durch Lagerbolzen gebildet werden. Die verschwenkbare Lagerung ist dabei so ausgebildet, dass das verschwenkbare Maulteil 4b über den Anlenkpunkt 9 mit der axial verschiebbaren Schaftstange 2 verbunden ist und eine Verlagerung dieser Schaftstange 2 ein Verschwenken des verschwenkbaren Maulteils 4b um den Anlenkpunkt 10 an der starren Schaftstange 1 bewirkt.

Durch Veränderung des Abstands der beiden Anlenkpunkte 9 und 10 des verschwenkbaren Maulteils 4b lassen sich so unterschiedliche Übersetzungsverhältnisse erzielen, um je nach Anwendungsgebiet des Instruments mehr oder weniger Kraft über die Maulteile 4a und 4b des Werkzeugs 4 ausüben zu können.

Da die Griffteile 5 und 6 aufgrund der Federelastizität immer wieder automatisch die dargestellte Ausgangsstellung einnehmen, in der das Werkzeug 4 sich in der Offenstellung befindet, kann es insbesondere bei der Ausbildung des Werkzeugs als Halteinstrument vorteilhaft sein, einen Arretiermechanismus 11 vorzusehen, wie dieser in Fig. 3 dargestellt ist, über den die Griffteile 5, 6 der Handhabe 7 und/oder die Schaftstangen 1, 2 zumindest in einer Schließstellung des Werkzeugs 4 in ihrer jeweiligen Lage zueinander fixierbar sind. Bei der in Fig. 3 dargestellten Ausführungsform ist der Arretiermechanismus 11 als am verstellbaren Griffteil 6 gelagerte Sperrklinke 11a ausgebildet, die über eine Federelement 11b vorgespannt arretierend in eine an der starren Schaftstange 1 ausgebildete Rastzahnung 11c eingreift. Das Lösen des Arretiermechanismus 11 erfolgt bei der dargestellten Ausführungsform über eine als Wippe ausgebildete Zeigefingerauflage 12.

Um dem Operateur das Ergreifen der Handhabe 7 zu erleichtern und eine sichere und präzise Bedienung des medizinischen Schneid- und/oder Halteinstruments zu gewährleisten, ist gemäß der in Fig. 2 dargestellten Ausführungsform die Handhabe 7 im Bereich der Griffteil 5 und 6 abgeflacht ausgebildet.

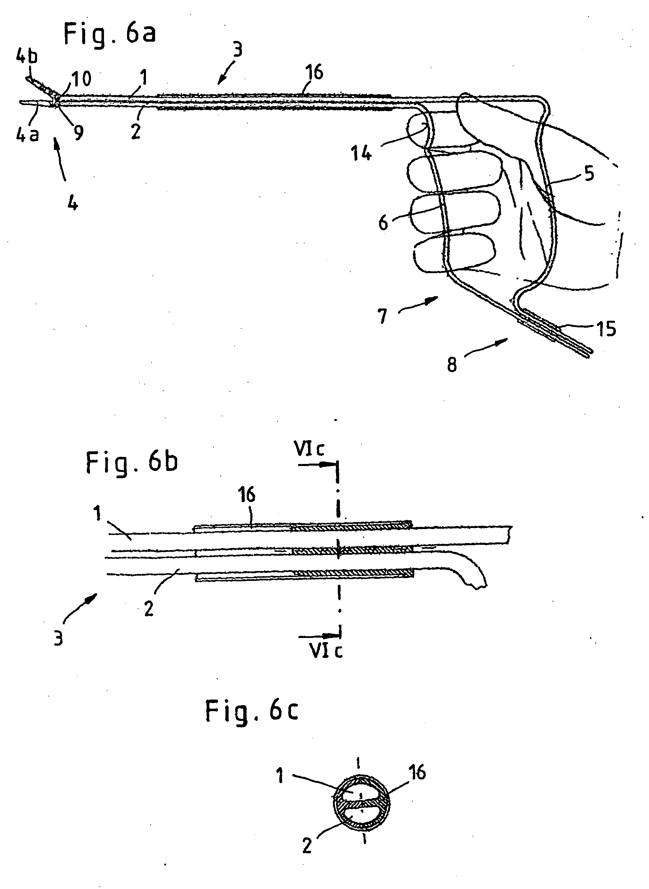

Die Abbildungen Fig. 4a und 6a zeigen alternative Ausführungsformen zur Verbesserung der Handhabbarkeit, wonach gemäß Fig. 4a an beiden Griffteilen 5 und 6 der Handhabe 7 eine Griffplatte 13 festgelegt ist und gemäß Fig. 6a an zumindest einem Griffteil 6 der Handhabe 7 mindestens eine Fingermulde 14 ausgebildet ist.

Zusätzlich zu der bereits zuvor beschriebenen Verbindung der Griffteile 5 und 6 der Handhabe 7 über das Kopplungselement 8 unterscheiden sich die in den Abbildungen Fig. 4a bis 6a dargestellten medizinischen Instrumente von den in den Abbildungen Fig. 1 bis 3 dargestellten Instrumenten dadurch, dass diese neben der Verwendung als bloße Schneid- und/oder Halteinstrumente auch so ausgestaltet sind, dass diese als monopolare oder bipolare Instrumente für die Elektrochirurgie verwendbar sind.

Bei der sogenannten Elektrochirurgie wird dem Operationsgebiet über das medizinische Instrument Hochfrequenzenergie zugeführt, die im Gewebe in Wärmeenergie umgewandelt wird, wobei am Umwandlungsort Temperaturen von etwa 60°C bis 100°C entstehen. Diese Elektrochirurgie wird zum Koagulieren von Gewebe und kleinen Gefäßen sowie zum Schneiden oder Trennen von Gewebe eingesetzt.

Zur Verwendung der dargestellten medizinischen Instrumente in der Elektrochirurgie sind die Griffteile 5 und 6 der Handhabe 7 mit einem Verbindungsstecker 15 verbunden sind, über den der Hochfrequenzstrom in das Instrument eingeleitet und bei der bipolaren Anwendung auch wieder abgeleitet wird. Bei den in den Abbildungen Fig. 4a bis 6a dargestellten Ausführungsformen ist der Verbindungsstecker 15 als die beiden Griffteile 5, 6 der Handhabe 7 miteinander verbindendes Kopplungselement 8 ausgebildet.

Bei der monopolaren Anwendung tritt die gewünschte thermische Wirkung nur an der sogenannten aktiven Elektrode, dem distalen Ende des medizinischen Instruments auf. Dieser Effekt wird dadurch bewirkt, dass die aktive Elektrode eine deutlich kleinere Kontaktfläche aufweist als die sogenannte neutrale Elektrode, auf der der Patient liegt. Der Stromfluss bei der monopolaren Hochfrequenz (HF)-Anwendung erfolgt ausgehend von einer HF-Stromquelle über den Verbindungsstecker 15 in das medizinische Instrument und weiter über die Griffteile 5 und 6 sowie die Schaftstangen 1 und 2 zu den Maulteilen 4a und 4b des Werkzeugs 4.

Bei Kontakt des Werkzeugs 4 mit dem Gewebe des Patienten schließt sich der Stromkreislauf und der gesamte, für die Wärmewirkung notwendige Hochfrequenzstrom fließt durch das Gewebevolümen hin zur neutralen Elektrode. Aufgrund der kleinen Kontaktfläche des als aktive Elektrode wirkenden Werkzeugs entwickelt sich nur in der Nähe des Werkzeugs eine hohe HF-Stromdichte und es findet auch nur in diesem Bereich eine Wärmeentwicklung mit entsprechend hoher Temperatur statt.

Bei der bipolaren Anwendung fließt der HF-Strom nur in einem Gewebevolumen zwischen zwei gleichartigen und gleichgroßen Elektroden, nämlich den Maulteilen 4a und 4b des Werkzeugs 4. Der Abstand zwischen den beiden Elektroden ist sehr gering und der HF-Strom fließt nur durch ein relativ kleines Gewebevolumen. Die Wärmeentwicklung findet ausschließlich zwischen den beiden Elektroden, den Maulteilen 4a und 4b statt. Der Stromfluss bei der bipolaren Hochfrequenz (HF)-Anwendung erfolgt ausgehend von einer HF-Stromquelle über den Verbindungsstecker 15 in das medizinische Instrument und weiter über den starren Griffteil 5 und die starre Schaftstange 1 zum verschwenkbaren Maulteil 4b des Werkzeugs 4. Sobald beide Maulteile 4a und 4b mit dem Gewebe des Patienten in Kontakt stehen, schließt sich der Stromkreislauf und der HF-Strom fließt über das starre Maulteil 4a des Werkzeugs 4 weiter über die axial verschiebbare Schaftstange 2 und den verlagerbaren Griffteil 6 der Handhabe 7 zurück über den Verbindungsstecker 15 zur HF-Stromquelle.

Zum Schutz des Operateurs sowie zum Schutz nicht zu behandelnder Gewebeteile sind bis auf die beiden Maulteile 4a und 4b des Werkzeugs 4 alle stromführenden Teile elektrisch isoliert ausgebildet. Bei der in Fig. 6a bis 6c dargestellten Ausführungsform ist die Isolierung als Schrumpfschlauch 16 ausgebildet, der die zu isolierenden Teile vollständig ummantelt, wie dies insbesondere den Schnittdarstellungen gemäß Fig. 6b und 6c zu entnehmen ist. Bei den Darstellungen ist exemplarisch nur die Isolierung der Schaftstangen 1 und 2 des Schaftes 3 dargestellt, obwohl in der Praxis auch die Griffteile 5 und 6 der Handhabe 7 isoliert ausgebildet sind.

Wie aus den Abbildungen ersichtlich, bieten die getrennt voneinander verlaufenden Schaftstangen 1 und 2 mit den einstückig gelenklos daran angeformten Griffteilen 5 und 6 der Handhabe 7 ausgesprochen günstige Isolationsbedingungen bei der Nutzung als Zu- und Ableitung des elektrischen Hochfrequenzstroms.

Um eine vorzeitige Überleitung des Stroms in die stromableitende Schaftstange 2 zu verhindern, ist bei der in Fig. 4a bis 4d dargestellten Ausführungsform das verschwenkbare Maulteil 4b über eine elektrisch isolierte, bzw. eine aus einem elektrisch nicht leitenden Material bestehende Gelenklasche 17 am starren Maulteil 4a und an der starren Schaftstange 1 gelagert. Wie insbesondere aus den Detailansichten gemäß Fig. 4c und 4d ersichtlich, ist die Gelenklasche 17 in einem Längsschlitz 18 des verschwenkbaren Maulteils 4b angeordnet, wobei der HF-Strom vorzugsweise von der starren Schaftstange 1 über den Lagerbolzen des Anlenkpunktes 10 in das verschwenkbare Maulteil 4b geleitet wird.

Die wie dargestellt ausgebildeten medizinischen Schneid- und/oder Halteinstrumente zeichnen sich zusätzlich zu einer vielseitigen Verwendung dadurch aus, dass sie aus nur wenigen Bauteilen bestehend einfach aufgebaut. Neben dem Effekt, dass derartige Instrumente einfach und schnell und somit kostengünstig zu fertigen und zu montieren sind, zeichnen sie sich durch eine gute Reinigbarkeit aus.

### Bezugszeichenliste

- 1: Schaftstange
- 2: Schaftstange
- 3: Schaft
- 4: Werkzeug
- 4a: starres Maulteil
- 4b: verschwenkbares Maulteil
- 5: Griffteil
- 6: Griffteil
- 7: Handhabe
- 8: Kopplungselement
- 9: Anlenkpunkt
- 10: Anlenkpunkt
- 11: Arretiermechanismus
- 11a: Sperrklinke
- 11b: Federelement
- 11c: Rastzahnung
- 12: Zeigefingerauflage
- 13: Griffplatte
- 14: Fingermulde
- 15: Verbindungsstecker
- 16: Schrumpfschlauch
- 17: Gelenklasche
- 18: Längsschlitz

## Patentansprüche

1. Medizinisches Schneid- und/oder Halteinstrument mit einem Schaft (3), einem am distalen Ende des Schaftes (3) angeordneten, aus zwei Maulteilen (4a, 4b) bestehenden Werkzeug (4) sowie mit einer am proximalen Ende des Schaftes (3) angeordneten, aus zwei Griffteilen (5, 6) bestehenden Handhabe (7) zum Öffnen und Schließen der Maulteile (4a, 4b) des Werkzeugs (4), wobei der Schaft (3) aus zwei jeweils einstückig gelenklos mit je einem der Griffteile (5, 6) der Handhabe (7) ausgebildeten Schaftstangen (1, 2) besteht,
**dadurch gekennzeichnet,**
**dass** das Werkzeug aus einem starren Maulteil (4a) und einem verschwenkbaren Maulteil (4b) besteht
und die Griffteile (5, 6) der Handhabe (7) federelastisch relativ zueinander verlagerbar sind.

2. Medizinisches Schneid- und/oder Halteinstrument nach Anspruch 1, **dadurch gekennzeichnet, dass** die Griffteile (5, 6) der Handhabe (7) über ein Kopplungselement (8) miteinander verbunden sind.

3. Medizinisches Schneid- und/oder Halteinstrument nach Anspruch 1, **dadurch gekennzeichnet, dass** die die beiden Griffteile (5, 6) aufweisende Handhabe (7) als einstückiges Bauteil ausgebildet ist.

4. Medizinisches Schneid- und/oder Halteinstrument nach einem der Ansprüche 1 bis 3, **dadurch gekennzeichnet, dass** die Handhabe (7) zumindest im Bereich eines Griffteils (5 oder 6) abgeflacht ausgebildet ist.

5. Medizinisches Schneid- und/oder Halteinstrument nach einem der Ansprüche 1 bis 3, **dadurch gekennzeichnet, dass** an mindestens einem Griffteil (5 oder 6) der Handhabe (7) mindestens eine Fingermulde (14) ausgebildet ist.

6. Medizinisches Schneid- und/oder Halteinstrument nach einem der Ansprüche 1 bis 3, **dadurch gekennzeichnet, dass** an mindestens einem Griffteil (5 oder 6) der Handhabe (7) eine Griffplatte (13) festlegbar ist.

7. Medizinisches Schneid- und/oder Halteinstrument nach einem der Ansprüche 1 bis 6, **dadurch gekennzeichnet, dass** ein Maulteil (4a) des Werkzeugs (4) einstückig starr mit einer Schaftstange (2) ausgebildet ist und das andere Maulteil (4b) um Anlenkpunkte (9, 10) verschwenkbar an der anderen Schaftstange (1) sowie am starren Maulteil (4a) gelagert ist.

8. Medizinisches Schneid- und/oder Halteinstrument nach Anspruch 7, **dadurch gekennzeichnet, dass** der Abstand der Anlenkpunkte (9 und 10) des verschwenkbaren Maulteils (4b) veränderbar ist.

9. Medizinisches Schneid- und/oder Halteinstrument nach einem der Ansprüche 1 bis 8, **dadurch gekennzeichnet, dass** die Griffteile (5, 6) der Handhabe (7) und/oder die Schaftstangen (1, 2) zumindest in einer Schließstellung des Werkzeugs (4) über einen Arretiermechanismus (11) in ihrer jeweiligen Lage zueinander fixierbar sind.

10. Medizinisches Schneid- und/oder Halteinstrument nach einem der Ansprüche 1 bis 9 zur Verwendung als monopolares oder bipolares Instrument in der Elektrochirurgie, **dadurch gekennzeichnet, dass** die Griffteile (5, 6) der Handhabe (7) mit einem Verbindungsstecker (15) verbunden sind.

11. Medizinisches Schneid- und/oder Halteinstrument nach Anspruch 10, **dadurch gekennzeichnet, dass** der Verbindungsstecker (15) als die beiden Griffteile (5, 6) der Handhabe (7) miteinander verbindendes Kopplungselement (8) ausgebildet ist.

12. Medizinisches Schneid- und/oder Halteinstrument nach Anspruch 10 oder 11, **dadurch gekennzeichnet, dass** bis auf die beiden Maulteile (4a, 4b) des Werkzeugs (4) alle stromführenden Teile; insbesondere mittels eines Schrumpfschlauches (16), elektrisch isoliert ausgebildet sind.

13. Medizinisches Schneid- und/oder Halteinstrument nach einem der Ansprüche 10 bis 12, **dadurch gekennzeichnet, dass** das verschwenkbare Maulteil (4b) über eine elektrisch isolierte Gelenklasche (17) am starren Maulteil (4a) und an der Schaftstange (1) gelagert ist.

## Claims

1. Medical cutting and/or holding instrument with a shaft (3), with a tool (4) arranged at the distal end of the shaft (3) and composed of two jaw parts (4a, 4b), and with a handle (7) which is arranged at the proximal end of the shaft (3), is composed of two grip parts (5, 6) and is used to open and close the jaw parts (4a, 4b) of the tool (4), the shaft (3) being composed of two shaft rods (1, 2) that are each formed hingelessly and in one piece with one of the grip parts (5, 6) of the handle (7), **characterized in that** the tool is composed of a rigid jaw part (4a) and a pivotable jaw part (4b), and the grip parts (5, 6) of the handle (7) can be moved relative to each other in a resilient manner.

2. Medical cutting and/or holding instrument according to Claim 1, **characterized in that** the grip parts (5, 6) of the handle (7) are connected to each other by a coupling element (8).

3. Medical cutting and/or holding instrument according to Claim 1, **characterized in that** the handle (7) with the two grip parts (5, 6) is designed as a one-piece component.

4. Medical cutting and/or holding instrument according to one of Claims 1 to 3, **characterized in that** the handle (7) has a flattened shape at least in the area of one grip part (5 or 6).

5. Medical cutting and/or holding instrument according to one of Claims 1 to 3, **characterized in that** at least one finger groove (14) is formed on at least one grip part (5 or 6) of the handle (7).

6. Medical cutting and/or holding instrument according to one of Claims 1 to 3, **characterized in that** a grip plate (13) can be secured on at least one grip part (5 or 6) of the handle (7).

7. Medical cutting and/or holding instrument according to one of Claims 1 to 6, **characterized in that** one jaw part (4a) of the tool (4) is formed rigidly in one piece with one shaft rod (2), and the other jaw part (4b) is mounted so as to pivot about articulation points (9, 10) on the other shaft rod (1) and on the rigid jaw part (4a).

8. Medical cutting and/or holding instrument according to Claim 7, **characterized in that** the distance between the articulation points (9 and 10) of the pivotable jaw part (4b) can be modified.

9. Medical cutting and/or holding instrument according to one of Claims 1 to 8, **characterized in that** the grip parts (5, 6) of the handle (7) and/or the shaft rods (1, 2), at least in a closed position of the tool (4), can be fixed in their respective position to each other by a locking mechanism (11).

10. Medical cutting and/or holding instrument according to one of Claims 1 to 9 for use as a monopolar or bipolar instrument in electrosurgery, **characterized in that** the grip parts (5, 6) of the handle (7) are connected to a connector plug (15).

11. Medical cutting and/or holding instrument according to Claim 10, **characterized in that** the connector plug (15) is designed as a coupling element (8) that connects the two grip parts (5, 6) of the handle (7) to each other.

12. Medical cutting and/or holding instrument according to Claim 10 or 11, **characterized in that**, except for the two jaw parts (4a, 4b) of the tool (4), all the current-conducting parts are electrically insulated, in particular by means of a heat-shrinkable sleeve (16).

13. Medical cutting and/or holding instrument according to one of Claims 10 to 12, **characterized in that** the pivotable jaw part (4b) is mounted on the rigid jaw part (4a) and on the shaft rod (1) via an electrically insulated joint bracket (17).

## Revendications

1. Instrument médical de coupe et/ou de maintien comportant une tige (3), un outil (4) disposé à l'extrémité distale de la tige (3), constitué de deux parties mors (4a, 4b), ainsi qu'une poignée (7) disposée à l'extrémité proximale de la tige (3), constituée de deux parties branches (5, 6), destinée à ouvrir et fermer les parties mors (4a, 4b) de l'outil (4), étant entendu que la tige (3) est constituée de deux corps de tige (1, 2) conçus monoblocs non articulés chacun avec une des parties branches (5, 6) de la poignée (7), **caractérisé en ce que** l'outil se compose d'une partie mors (4a) rigide et d'une partie mors (4b) pivotable et les parties branches (5, 6) de la poignée (7) sont déplaçables l'une par rapport à l'autre par déformation élastique.

2. Instrument médical de coupe et/ou de maintien selon la revendication 1, **caractérisé en ce que** les parties branches (5, 6) de la poignée (7) sont reliées entre elles par un élément de couplage (8).

3. Instrument médical de coupe et/ou de maintien selon la revendication 1, **caractérisé en ce que** la poignée (7) présentant les deux parties branches (5, 6) est conçue sous forme de composant monobloc.

4. Instrument médical de coupe et/ou de maintien selon une des revendications 1 à 3, **caractérisé en ce que** la poignée (7) est conçue aplatie au moins dans la région d'une partie branche (5 ou 6).

5. Instrument médical de coupe et/ou de maintien selon une des revendications 1 à 3, **caractérisé en ce qu'**au moins une dépression pour le doigt (14) est conçue sur au moins une partie branche (5 ou 6) de la poignée (7).

6. Instrument médical de coupe et/ou de maintien selon une des revendications 1 à 3, **caractérisé en ce qu'**une plaque de branche (13) peut être fixée à au moins une partie branche (5 ou 6) de la poignée (7).

7. Instrument médical de coupe et/ou de maintien selon une des revendications à 6, **caractérisé en ce qu'**une partie mors (4a) de l'outil (4) est conçue monobloc et rigide avec un corps de tige (2) et l'autre partie mors (4b) est logée de façon à pouvoir pivoter autour de points d'articulation (9, 10) sur l'autre corps de tige (1) ainsi que sur la partie mors rigide (4a).

8. Instrument médical de coupe et/ou de maintien selon la revendication 7, **caractérisé en ce que** la distance entre les points d'articulation (9 et 10) de la partie mors pivotable (4b) est modifiable.

9. Instrument médical de coupe et/ou de maintien selon une des revendications 1 à 8, **caractérisé en ce que** les parties branches (5, 6) de la poignée (7) et/ou les corps de tige (1, 2) sont immobilisables dans leur situation respective au moins dans une position de fermeture de l'outil (4) par l'intermédiaire d'un mécanisme d'arrêt (11).

10. Instrument médical de coupe et/ou de maintien selon une des revendications 1 à 9 pour utilisation comme instrument monopolaire ou bipolaire en électrochirurgie, **caractérisé en ce que** les parties branches (5, 6) de la poignée (7) sont reliées par une fiche de connexion (15).

11. Instrument médical de coupe et/ou de maintien selon la revendication 10, **caractérisé en ce que** la fiche de connexion (15) est conçue comme élément de couplage (8) reliant entre elles les deux parties branches (5, 6) de la poignée (7).

12. Instrument médical de coupe et/ou de maintien selon la revendication 10 ou 11, **caractérisé en ce que**, à l'exception des deux parties mors (4a, 4b) de l'outil (4), toutes les parties conductrices sont conçues isolées électriquement, en particulier au moyen d'une gaine thermorétractable (16).

13. Instrument médical de coupe et/ou de maintien selon une des revendications 10 à 12, **caractérisé en ce que** la partie mors pivotable (4b) est logée par l'intermédiaire d'une attache articulée isolée électriquement (17) sur la partie mors rigide (4a) et sur le corps de tige (1).
